(19) Europäisches Patentamt
European Patent Office
Office européen des brevets

(11) EP 3 318 173 A1

(12) **EUROPEAN PATENT APPLICATION**
published in accordance with Art. 153(4) EPC

(43) Date of publication:
09.05.2018 Bulletin 2018/19

(51) Int Cl.:
*A61B 1/00* (2006.01)  *A61B 1/04* (2006.01)
*G01S 17/89* (2006.01)  *G02B 23/24* (2006.01)

(21) Application number: 16817498.5

(22) Date of filing: 17.02.2016

(86) International application number:
PCT/JP2016/054621

(87) International publication number:
WO 2017/002388 (05.01.2017 Gazette 2017/01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 30.06.2015 JP 2015131904

(71) Applicant: Olympus Corporation
Tokyo 192-8507 (JP)

(72) Inventor: SATO, Daisuke
Hachioji-shi
Tokyo 192-8507 (JP)

(74) Representative: Gunzelmann, Rainer
Wuesthoff & Wuesthoff
Patentanwälte PartG mbB
Schweigerstraße 2
81541 München (DE)

(54) **IMAGE PROCESSING DEVICE, RANGING SYSTEM, AND ENDOSCOPE SYSTEM**

(57) Provided is an image processing apparatus, or the like, capable of obtaining high-accuracy depth information without drastically increasing the data processing amount on an imaging unit and data communication amount with the imaging unit. The image processing apparatus is configured to perform image processing based on image data representing an image of a subject and ranging data representing a distance to the subject output from the imaging unit. The image processing apparatus includes: an illumination light distribution characteristic calculation unit 332 that calculates a parameter of illumination light emitted onto the subject based on the ranging data; a reflected light distribution characteristic calculation unit 334, that calculates a parameter of reflected light of the illumination light reflected from the subject, based on a gradient of a depth on a point on the subject calculated from the ranging data; and a subject distance calculation unit 340 that calculates a depth from the imaging unit to the subject in an optical axis direction of a collection optical system, based on the image data, the parameter of the illumination light calculated the illumination light distribution characteristic calculation unit 332, and the parameter of the reflected light calculated by the reflected light distribution characteristic calculation unit 334.

FIG.3

EP 3 318 173 A1

Printed by Jouve, 75001 PARIS (FR)

**Description**

Field

The present invention relates to an image processing apparatus for performing image processing based on data obtained by imaging an inside of a living body. The present invention also relates to a ranging system and an endoscope system.

Background

Endoscope systems have been widely used to perform diagnosis of the living body by introducing an endoscope into a living body to observe images of a subject captured by the endoscope. In recent years, endoscope systems incorporating a ranging system for measuring a distance (depth) from the endoscope to the subject have been developed.
As an exemplary ranging system, Patent Literature 1 discloses a system that includes, in an imaging unit, an image sensor for image plane phase difference detection auto-focus (AF), and that measures the depth to the subject on the basis of an output signal from a ranging pixel disposed on the image sensor.
Moreover, Patent Literature 2 discloses a system that includes, in an imaging unit, a time of flight (TOF)-system ranging sensor independently of the image sensor for generating images of the subject, and that measures the depth to the subject on the basis of the output signal from the ranging sensor.
Furthermore, Patent Literature 3 discloses a technique of calculating the depth from the image of the subject on the basis of the positional relationship between the illumination unit that illuminates the subject, and the imaging unit. Specifically, the depth to the subject is calculated using an emission angle (angle with respect to the optical axis of the illumination unit) of the light emitted from the illumination unit and incident on a point of interest on the subject, and using an imaging angle (angle with respect to the optical axis of collection optical system) of the light that is reflected from the point of interest and incident on the imaging unit via the collection optical system.

Citation List

Patent Literature

    Patent Literature 1: JP 2013-232751 A
    Patent Literature 2: JP 2009-267436 A
    Patent Literature 3: JP 2009-41929 A

Summary

Technical Problem

Enhancing accuracy in measuring the depth to the subject on the image is important in grasping a stereoscopic structure of the subject and in calculating a distance between two points on the subject corresponding to the two points within the image using the principles of triangulation.
In Patent Literature 1, however, since merely the depth at a position of each of the ranging pixels sparsely arranged on the image sensor is actually measured, it is not possible to obtain high-accuracy depth information for all the pixel positions included in the image of the subject.
On the other hand, since the technique in Patent Literature 2 actually measures the depth in a whole region within the image, the amount of data generated for one frame is drastically increased even though high-accuracy depth information can be obtained. Accordingly, it takes a long time to perform data processing on the ranging sensor and to perform data communication from the ranging sensor to the image processing apparatus. This would result in an issue of reduction of the imaging frame rate.
Furthermore, according to Patent Literature 3, since the emission angle cannot be obtained from the image of the subject, there would be no other way than using an estimated value. Therefore, the accuracy in measuring the depth would depend upon the estimated accuracy of the emission angle, leading to a possible failure in acquiring high-accuracy depth information.
The present invention has been made in view of the foregoing, and an object of the present invention is to provide an image processing apparatus, a ranging system, and an endoscope system that are capable of obtaining high-accuracy depth information without drastically increasing data processing amount on the imaging unit and data communication amount between the imaging unit and the image processing apparatus.

Solution to Problem

In order to solve the above described problem and achieve the object, an image processing apparatus according to the invention is configured to perform image processing based on image data and ranging data output from an imaging unit. The imaging unit includes: an illumination unit configured to generate illumination light to be emitted onto a subject; a collection optical system configured to collect the illumination light reflected from the subject; and an image sensor configured to receive the illumination light collected by the collection optical system, and output the image data representing an image of the subject and output the ranging data representing a distance to the subject, based on the illumination light. The image processing apparatus includes: an illumination light distribution characteristic calculation unit configured to calculate a parameter of the illumination light emitted onto a point on the subject, based on the ranging data; a reflected light distribution characteristic calculation unit configured to calculate a parameter of reflected light of the illumination light reflected from the subject, based on a gradient of a depth on the point on the subject calculated from the ranging data; and a subject distance calculation unit configured to calculate the distance between the imaging unit and the subject in an optical axis direction of the collection optical system, based on the image data, the parameter of the illumination light, and the parameter of the reflected light.

The image processing apparatus further includes a depth image creation unit configured to create, based on the ranging data, a depth image in which the depth to the point on the subject corresponding to each of pixel positions of an image of the subject created based on the image data is defined as a pixel value of each pixel. The illumination light distribution characteristic calculation unit is configured to calculate a value of distribution characteristics of the illumination light in a radiation angle direction, based on the depth image.

In the image processing apparatus, the depth image creation unit is configured to perform interpolation on the depth at a pixel position where the ranging data has not been obtained, among the pixel positions of the image of the subject, using the ranging data at a pixel position where the ranging data has been obtained.

The image processing apparatus further includes a depth gradient calculation unit configured to calculate the gradient of the depth for each of the pixel positions of the image of the subject, based on the depth calculated by the depth image creation unit. The reflected light distribution characteristic calculation unit is configured to calculate a value of distribution characteristics of the reflected light in a reflection angle direction, based on the gradient of the depth calculated by the depth gradient calculation unit.

The image processing apparatus further includes: an image processing unit configured to create an image for display based on the image data; a display unit configured to display the image for display; an operation input unit configured to input a signal for designating any two points on the image for display; and a two-point distance calculation unit configured to calculate a distance between two points on the subject corresponding to any two points on the image for display.

A ranging system according to the invention includes the image processing apparatus and the imaging unit.

An endoscope system according to the invention includes the image processing apparatus, and a capsule endoscope including the imaging unit and a capsule-shaped casing that accommodates the imaging unit.

An endoscope system according to the invention includes: the image processing apparatus; and a video scope including an insertion unit configured to be inserted into the subject and including the imaging unit at a distal end portion of the insertion unit.

According to the invention, by using parameters of illumination light and reflected light calculated based on ranging data indicating a distance to a subject and using image data indicating an image of the subject, it is possible to calculate, with a high degree of accuracy, a depth between an imaging unit and the subject. With this feature, there is no need to actually measure depths for positions of all pixels constituting the image of the subject, which makes it possible to acquire high-accuracy depth information without drastically increasing data processing amount and data communication amount.

Brief Description of Drawings

FIG. 1 is a schematic diagram illustrating an exemplary configuration of a ranging system according to a first embodiment of the present invention.
FIG. 2 is a schematic diagram illustrating a light-receiving surface of an image sensor illustrated in FIG. 1.
FIG. 3 is a block diagram illustrating a configuration of a depth calculation unit illustrated in FIG. 1.
FIG. 4 is a schematic diagram for illustrating a principle of measuring a subject distance.
FIG. 5 is a schematic diagram for illustrating distribution characteristics of illumination light.

FIG. 6 is a schematic diagram for illustrating an image region of a depth image corresponding to the light-receiving surface of the image sensor illustrated in FIG. 4.

FIG. 7 is a schematic diagram for illustrating a method for calculating a depth gradient.

FIG. 8 is a schematic diagram for illustrating a method for calculating a depth gradient.

FIG. 9 is a schematic diagram for illustrating distribution characteristics of reflected light.

FIG. 10 is a schematic diagram illustrating an exemplary configuration of a ranging system according to a second embodiment of the present invention.

FIG. 11 is a schematic diagram for illustrating an exemplary screen displayed on a display unit illustrated in FIG. 10.

FIG. 12 is a schematic diagram for illustrating a principle of measuring a distance on a subject, corresponding to a distance between two points within the image.

FIG. 13 is a schematic diagram for illustrating a principle of measuring a distance on a subject, corresponding to a distance between two points within the image.

FIG. 14 is a schematic diagram illustrating an exemplary configuration of an endoscope system according to a third embodiment of the present invention.

FIG. 15 is a schematic diagram illustrating an exemplary internal structure of a capsule endoscope illustrated in FIG. 14.

FIG. 16 is a schematic diagram illustrating an exemplary configuration of an endoscope system according to a fourth embodiment of the present invention.

Description of Embodiments

Hereinafter, an image processing apparatus, a ranging system, and an endoscope system according to embodiments of the present invention will be described with reference to the drawings. The drawings merely schematically illustrate shapes, sizes, and positional relations to the extent that contents of the present invention are understandable. Accordingly, the present invention is not limited only to the shapes, sizes, and positional relations exemplified in the individual drawings. The same reference signs are used to designate the same elements throughout the drawings.

(First Embodiment)

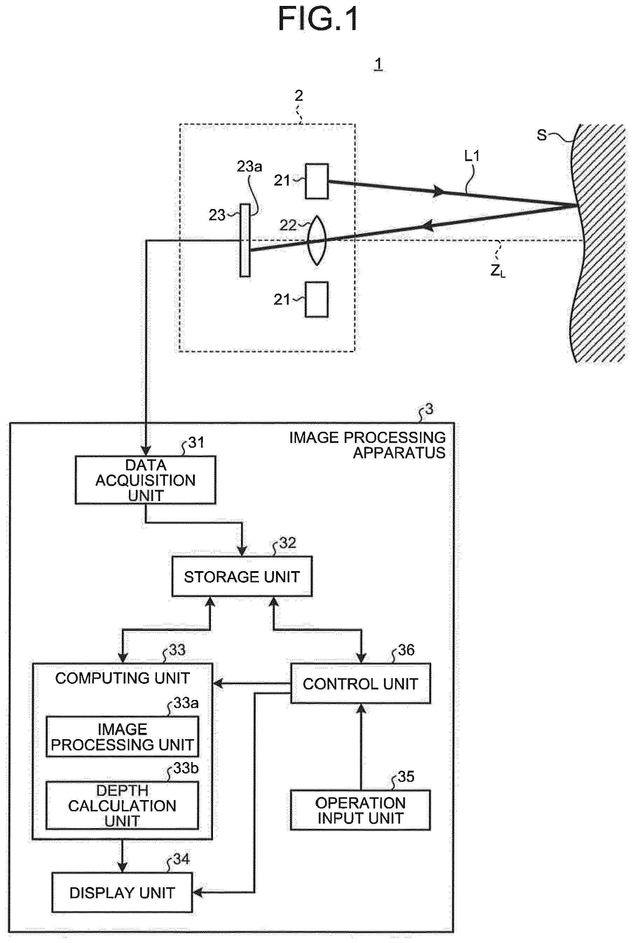

FIG. 1 is a schematic diagram illustrating an exemplary configuration of a ranging system according to a first embodiment of the present invention. A ranging system 1 according to the first embodiment is a system that is applied to an endoscope system, or the like, that is introduced into a living body to perform imaging and that measures the distance (depth) to a subject such as mucosa. The endoscope system may be a typical endoscope system having a video scope including an imaging unit at a distal end portion of an insertion unit inserted into the subject, or alternatively, may be a capsule endoscope system including a capsule endoscope configured to be introduced into the living body. The capsule endoscope includes an imaging unit and a wireless communication unit in a capsule-shaped casing, and is configured to perform image capturing.

As illustrated in FIG. 1, the ranging system 1 includes an imaging unit 2 that images a subject S, thereby generating and outputting image data, and actually measures the distance to the subject S, thereby generating and outputting ranging data, and includes an image processing apparatus 3 that obtains the image data and the ranging data output from the imaging unit 2 and creates an image of the subject S on the basis of the image data, as well as creates a depth map to the subject S using the image data and the ranging data.

The imaging unit 2 includes an illumination unit 21 configured to emit illumination light to irradiate a subject S, a collection optical system 22 such as a condenser lens, and an image sensor 23.

The illumination unit 21 includes a light emitting element such as a light emitting diode (LED), and a drive circuit for driving the light emitting element. The illumination unit 21 generates white light or illumination light with a specific frequency band and emits the light onto the subject S.

The image sensor 23 is a sensor capable of obtaining image data representing visual information on the subject S and ranging data representing the depth to the subject S. The image sensor 23 includes a light-receiving surface 23a that receives illumination light (namely, reflected light) emitted from the illumination unit 21, reflected from subject S, and collected by the collection optical system 22. In the first embodiment, an image plane phase difference detection AF sensor is employed as the image sensor 23.

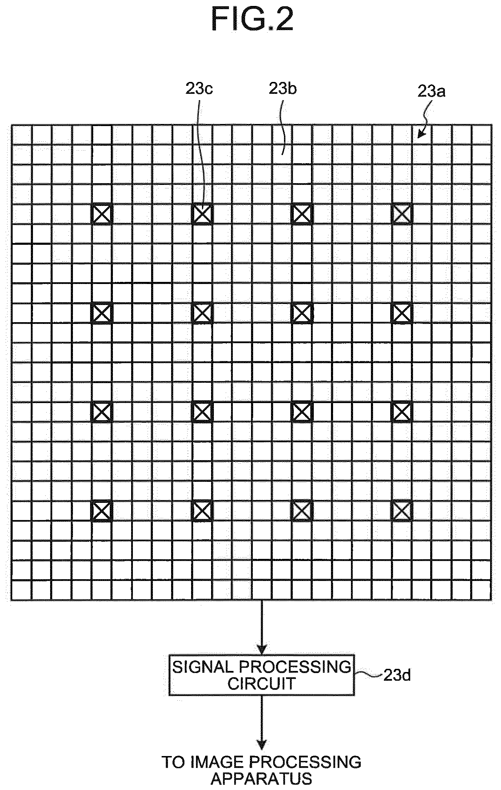

FIG. 2 is a schematic diagram for illustrating a configuration of the image sensor 23. As illustrated in FIG. 2, the image sensor 23 includes a plurality of imaging pixels 23b, ranging pixels 23c, and a signal processing circuit 23d. The plurality of imaging pixels is arranged on the light-receiving surface 23a. The signal processing circuit 23d processes an electrical signal output from these pixels. A plurality of imaging pixels 23b is arrayed in a matrix on the light-receiving surface 23a. A plurality of ranging pixels 23c is arranged so as to replace a portion of the matrix of the plurality of imaging pixels 23b. In FIG. 2, check marks are drawn for the positions of the ranging pixels 23c to be distinguished from the imaging pixels 23b.

Each of the imaging pixels 23b has a structure including a microlens and any of color filters red (R), green (G), and blue (B) stacked on a photoelectric conversion unit such as a photodiode, and generates an electric charge corresponding to the amount of light incident onto the photoelectric conversion unit. The imaging pixels 23b are arranged in a predetermined order, such as a Bayer array, in accordance with the color of the color filter included in each of the pixels. The signal processing circuit 23d converts the electric charge generated by each of the imaging pixels 23b into a voltage signal, and further converts the voltage signal into a digital signal, thereby outputting the signal as image data.

Each of the ranging pixels 23c has a structure in which two photoelectric conversion units are arranged on a same plane side by side and furthermore, one microlens is disposed so as to be placed across these photoelectric conversion units. The light incident onto the microlens is further incident onto the two photoelectric conversion units at the distribution ratio corresponding to the incident position at the microlens. Each of the two photoelectric conversion units generates an electric charge corresponding to the amount of incident light. The signal processing circuit 23d converts the electric charge generated at the two photoelectric conversion units of the ranging pixel 23c into a voltage signal, generates and outputs ranging data representing a distance (depth) from the imaging unit 2 to the subject S on the basis of the phase difference (information regarding distance) between these voltage signals.

The image processing apparatus 3 includes a data acquisition unit 31 that obtains image data and ranging data output from the imaging unit 2, a storage unit 32 that stores the image data and the ranging data obtained by the data acquisition unit 31 and stores various programs and parameters used on the image processing apparatus 3, a computing unit 33 that performs various types of calculation processing on the basis of the image data and the ranging data, a display unit 34 that displays an image of the subject S, or the like, an operation input unit 35 that is used for inputting various types of information and commands into the image processing apparatus 3, and a control unit 36 for performing overall control of these elements.

The data acquisition unit 31 is appropriately configured in accordance with a mode of the endoscope system to which the ranging system 1 is applied. For example, in the case of a typical endoscope system configured to insert a video scope into the body, the data acquisition unit 31 includes an interface that captures the image data and the ranging data generated by the imaging unit 2 provided at the video scope. Moreover, in the case of a capsule endoscope system, the data acquisition unit 31 includes a receiving unit that receives a signal wirelessly transmitted from a capsule endoscope via an antenna. Alternatively, image data and ranging data may be sent and received to and from the capsule endoscope, using a portable storage medium. In this case, the data acquisition unit 31 includes a reader apparatus that removably attaches the portable storage medium and reads out the stored image data and ranging data. Alternatively, in a case where a server to store the image data and the ranging data generated in the endoscope system is installed, the data acquisition unit 31 includes a communication apparatus, or the like, to be connected with the server, and obtains various types of data by performing data communication with the server.

The storage unit 32 includes an information storage apparatus including various types of integrated circuit (IC) memories such as renewable flash memories including a read only memory (ROM) and a random access memory (RAM), a hard disk that is either internal or connected with a data communication terminal, or a compact disc read only memory (CD-ROM), together with an apparatus for reading information from and writing information into the information storage apparatus. The storage unit 32 stores programs for operating the image processing apparatus 3 and causing the image processing apparatus 3 to execute various functions, data used in execution of the program, specifically, image data and ranging data obtained by the data acquisition unit 31, and various parameters.

The computing unit 33 includes a general-purpose processor such as a central processing unit (CPU), a dedicated processor including various calculation circuits such as an application specific integrated circuit (ASIC), for executing specific functions, or the like. In a case where the computing unit 33 is a general-purpose processor, it executes calculation processing by reading various calculation programs stored in the storage unit 32. In another case where the computing unit 33 is a dedicated processor, the processor may execute various types of calculation processing independently, or the processor may execute calculation processing in cooperation with or combined with the storage unit 32 using various data stored in the storage unit 32.

Specifically, the computing unit 33 includes an image processing unit 33a that performs predetermined image processing such as white balance processing, demosaicing, gamma conversion, smoothing (noise removal, etc.) on the image data, thereby generating image for display, and includes a depth calculation unit 33b that calculates a depth (distance from the collection optical system 22) to the subject S corresponding to each of the pixel positions within the image for display, created by the image processing unit 33a, on the basis of the image data and the ranging data The configuration and operation of the depth calculation unit 33b will be described in detail below.

The display unit 34 includes various types of displays formed of liquid crystal, organic electroluminescence (EL), or the like, and displays an image for display created by the image processing unit 33a, and information such as the distance calculated by the depth calculation unit 33b, or others.

The control unit 36 includes a general-purpose processor such as a CPU, and a dedicated processor including various calculation circuits such as an ASIC, for executing specific functions. In a case where the control unit 36 is a general-purpose processor, the processor performs overall control of the image processing apparatus 3, including transmission of an instruction and data to each element of the image processing apparatus 3, by reading a control program stored in the storage unit 32. In another case where the control unit 36 is a dedicated processor, the processor may execute various types of processing independently, or the processor may execute various types of processing in cooperation with or combined with the storage unit 32 using various types of data stored in the storage unit 32.

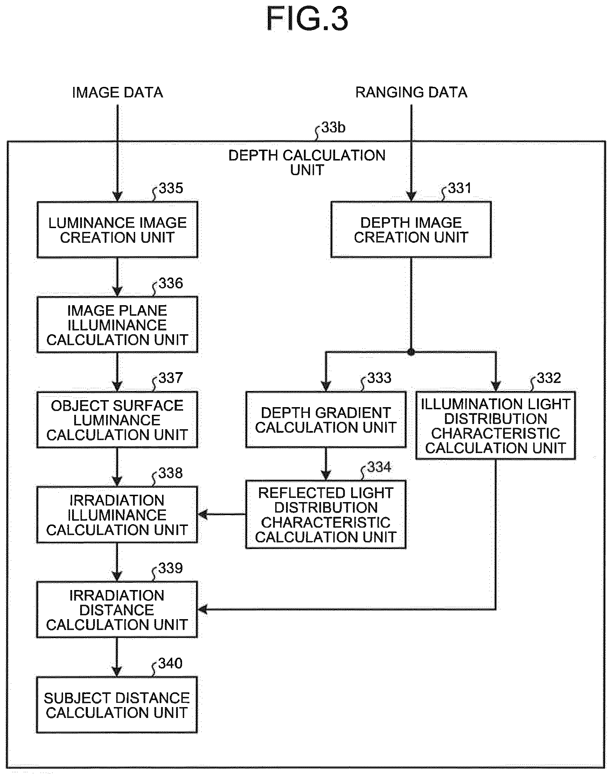

FIG. 3 is a block diagram illustrating a detailed configuration of the depth calculation unit 33b. As illustrated in FIG. 3, the depth calculation unit 33b includes a depth image creation unit 331, an illumination light distribution characteristic calculation unit 332, a depth gradient calculation unit 333, a reflected light distribution characteristic calculation unit 334, a luminance image creation unit 335, an image plane illuminance calculation unit 336, an object surface luminance calculation unit 337, an irradiation illuminance calculation unit 338, an irradiation distance calculation unit 339, and a subject distance calculation unit 340.

The depth image creation unit 331 creates a depth image in which the depth between a point on the subject S corresponding to each of the pixel positions within an image for display created by the image processing unit 33a and the collection optical system 22 is defined as a pixel value of each of the pixels, on the basis of the ranging data read from the storage unit 32. As described above, since the ranging pixels 23c are sparsely arranged on the light-receiving surface 23a, the depth image creation unit 331 calculates the depth for the pixel position on which the ranging pixel 23c is not disposed, by interpolation calculation using the ranging data output from the ranging pixel 23c disposed in vicinity.

The illumination light distribution characteristic calculation unit 332 calculates a value in a radiation angle direction on the light distribution characteristic, as a parameter of the illumination light emitted onto the subject S, on the basis of the depth image created by the depth image creation unit 331.

The depth gradient calculation unit 333 calculates a gradient of depth (depth gradient) on a point of the subject S on the basis of the depth image created by the depth image creation unit 331.

The reflected light distribution characteristic calculation unit 334 calculates a value in a reflection angle direction on the light distribution characteristic, as a parameter of the illumination light reflected from the subject S (that is, reflected light), on the basis of the depth gradient calculated by the depth gradient calculation unit 333.

The luminance image creation unit 335 creates a luminance image defining the luminance of the image of the subject S as a pixel value of each of the pixels on the basis of the image data read from the storage unit 32.

The image plane illuminance calculation unit 336 calculates illuminance on the image plane of the image sensor 23 on the basis of the luminance image created by the luminance image creation unit 335.

The object surface luminance calculation unit 337 calculates the luminance on the surface of the subject S on the basis of the illuminance on the image plane calculated by the image plane illuminance calculation unit 336.

The irradiation illuminance calculation unit 338 calculates irradiation illuminance of the illumination light emitted onto the subject S, on the basis of the luminance of the object surface calculated by the object surface luminance calculation unit 337 and on the basis of the value in the reflection angle direction of the distribution characteristics of the reflected light calculated by the reflected light distribution characteristic calculation unit 334.

The irradiation distance calculation unit 339 calculates an irradiation distance from the collection optical system 22 to the subject S on the basis of the irradiation illuminance of the illumination light emitted onto the subject S and on the basis of the value in the radiation angle direction of the distribution characteristics of the illumination light calculated by the illumination light distribution characteristic calculation unit 332.

The subject distance calculation unit 340 calculates a subject distance, that is, an irradiation distance calculated by the irradiation distance calculation unit 339, projected onto an optical axis $Z_L$ of the collection optical system 22.

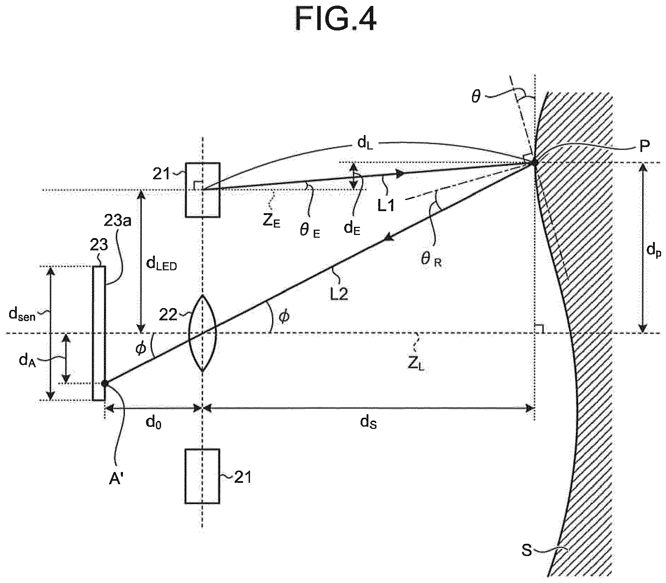

Next, a ranging method according to the first embodiment will be described in detail with reference to FIGS. 1 to 8. FIG. 4 is schematic diagram for illustrating positional and angular relationships between the subject S and each element of the imaging unit 2.

First, the ranging system 1 emits illumination light L1 onto the subject S by causing the illumination unit 21 to emit light. With this process, the illumination light reflected from the subject S (that is, reflected light) is collected by the collection optical system 22 and becomes incidence on the light-receiving surface 23a of the image sensor 23. On the basis of an electric signal output individually from the imaging pixel 23b and the ranging pixel 23c arranged on the light-receiving surface 23a, the signal processing circuit 23d (refer to FIG. 2) outputs image data for the position on each of the imaging pixels 23b, as well as outputs ranging data for the position of each of the ranging pixels 23c. The data acquisition unit 31 of the image processing apparatus 3 captures the image data and the ranging data and stores the data in the storage unit 32.

As illustrated in FIG. 3, the depth calculation unit 33b captures the ranging data and the image data from the storage unit 32, then, inputs the ranging data into the depth image creation unit 331, while inputting the image data into the luminance image creation unit 335.

The depth image creation unit 331 creates a depth image of a size corresponding to the entire light-receiving surface 23a by defining a distance $d_S$ (refer to FIG. 4) from the collection optical system 22 to the subject S as a pixel value of each of the pixels on the basis of the input ranging data. As illustrated in FIG. 2, the ranging pixels 23c are sparsely arranged on the light-receiving surface 23a of the image sensor 23. Accordingly, the depth image creation unit 331 calculates a value using ranging data based on an output value from the ranging pixel 23c for the pixel within the depth image that is corresponding to the position of the ranging pixel 23c, and calculates a value by interpolation using ranging data for other pixels within the depth image. Accordingly, the distance $d_S$ on the depth image, on the pixel position for which a measurement value based on the output value from the ranging pixel 23c has not been obtained, is an approximate value that has not received any feedback on irregularities of the surface of the subject S, or the like.

Subsequently, on the basis of the depth image created by the depth image creation unit 331, the illumination light distribution characteristic calculation unit 332 calculates a value in the radiation angle direction of the distribution characteristics of the illumination light L1 emitted onto each of the points (e.g. a point of interest P) on the subject S.

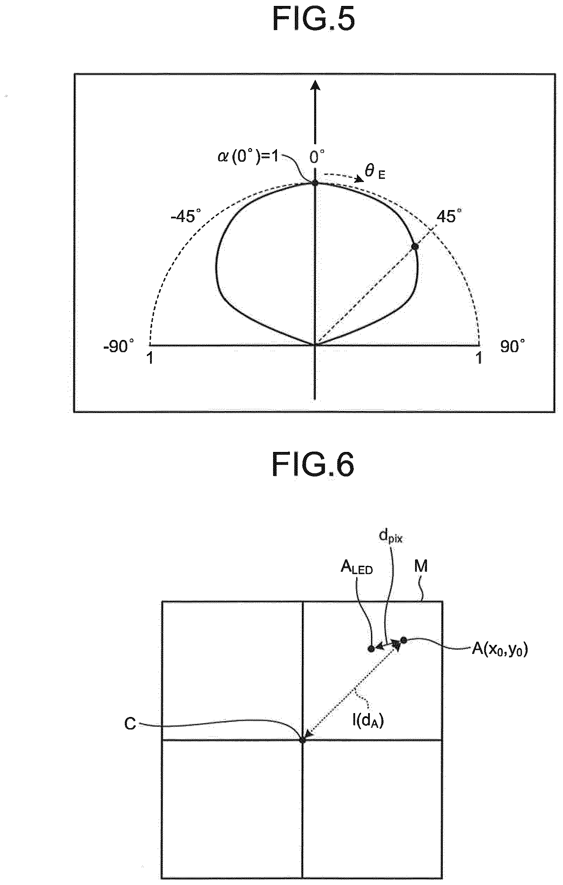

FIG. 5 illustrates a relationship between a radiation angle $\theta_E$ formed by the radiation direction of the illumination light L1 with respect to an optical axis $Z_E$ of the illumination unit 21, and a value $\alpha(\theta_E)$ in the radiation angle direction on the light distribution characteristic corresponding to the radiation angle $\theta_E$. In FIG. 5, normalization is performed on the basis of the maximum light intensity on a radiation surface, that is, the light intensity when the radiation angle $\theta_E = 0°$. The illumination light distribution characteristic calculation unit 332 reads, from the storage unit 32, a function or a table indicating light distribution characteristic illustrated in FIG. 5, calculates the radiation angle $\theta_E$ from the positional relationship between the illumination unit 21 and the point of interest P, and calculates the value $\alpha(\theta_E)$ in the radiation angle direction on the light distribution characteristic corresponding to the radiation angle $\theta_E$.

A typical LED has light distribution characteristics represented in cosine, and thus, in a case where the radiation angle $\theta_E = 45°$, light intensity $\alpha(45°)$ in the radiation angle direction is obtained by multiplying the value $\alpha(0°)$ at the radiation angle $\theta_E = 0°$ by $\cos(45°)$.

Now a method for calculating the radiation angle $\theta_E$ will be described. FIG. 6 is a schematic diagram illustrating an image region of a depth image corresponding to the light-receiving surface 23a of the image sensor 23. First, the illumination light distribution characteristic calculation unit 332 extracts a pixel A' (refer to FIG. 4) on the light-receiving surface 23a, that corresponds to a pixel of interest A $(x_0, y_0)$ within a depth image M, and converts the coordinate value of the pixel A' from pixel into distance (mm) using the number of pixels of the image sensor 23 and a sensor size $d_{sen}$ (mm). Moreover, the illumination light distribution characteristic calculation unit 332 calculates a distance from the optical axis $Z_L$ of the collection optical system 22 to the pixel A', namely, an image height $d_A$ using the coordinate value of the pixel A' that has been converted into the distance. Then, a field angle $\phi$ is calculated by the following formula (1) on the basis of a distance $(d_0)$ (design value) from the collection optical system 22 to the light-receiving surface 23a, and the image height $d_A$.

$$\phi = \tan^{-1}(d_A/d_0) \qquad \ldots (1)$$

A length $l(d_A)$ within the depth image M, corresponding to the image height $d_A$, is indicated in a broken line in FIG. 6.

Using the following formula (2), the illumination light distribution characteristic calculation unit 332 calculates a distance between the point of interest P on the subject S corresponding to the pixel of interest A, and the optical axis $Z_L$, namely, a height $d_P$ of the subject, on the basis of the field angle $\phi$, the pixel value of the pixel of interest A on the depth image M, namely, a depth $d_S$.

$$d_P = d_S \tan\phi \qquad \ldots (2)$$

Subsequently, the illumination light distribution characteristic calculation unit 332 calculates the coordinate within the depth image M, corresponding to the position of the light emitting element included in the illumination unit 21. On the imaging unit 2, a distance $d_{LED}$ between the optical axis $Z_E$ of the light emitting element included in the illumination unit 21, and the optical axis $Z_L$ of the collection optical system 22, is determined as a design value; and also the positional relationship between the light emitting element and the light-receiving surface 23a of the image sensor 23 is determined as a design value. Accordingly, the illumination light distribution characteristic calculation unit 332 obtains an image height of the depth image M using the number of pixels and the sensor size $d_{sen}$ (mm) of the image sensor 23, and calculates a coordinate $A_{LED}$ of the pixel within the depth image M, corresponding to the position of the light emitting element included in the illumination unit 21 on the basis of the obtained image height.

Subsequently, the illumination light distribution characteristic calculation unit 332 calculates an interval $D_{pix}$ of these pixels on the basis of the coordinate of the pixel of interest A and from the coordinate of a pixel $A_{LED}$ corresponding to the position of the light emitting element. Then, the interval $d_{pix}$ is converted into the distance (mm) on the subject S using the number of pixels and the sensor size $d_{sen}$ (mm) of the image sensor 23. This distance is a distance $d_E$ from the point of interest P to the optical axis $Z_E$ of the light emitting element. Using the following formula (3), the illumination light distribution characteristic calculation unit 332 calculates the radiation angle $\theta_E$ on the basis of the distance $d_E$ and the depth $d_S$ of the point of interest P.

$$\theta_E = \tan^{-1}(d_E/d_S) \qquad \qquad \ldots (3)$$

On the basis of the radiation angle $\theta_E$ calculated in this manner, the illumination light distribution characteristic calculation unit 332 calculates a value $\alpha(\theta_E)$ (FIG. 5) in the radiation angle direction of the distribution characteristics of the illumination light L1.

If the illumination unit 21 has a plurality of light emitting elements, the illumination light distribution characteristic calculation unit 332 may calculate, for each of the plurality of light emitting elements, the radiation angle $\theta_E$ using the above-described technique, and calculate a value of the light distribution characteristics in the radiation angle direction based on the calculated plurality of radiation angles $\theta_E$. In this case, a function or a table representing characteristics corresponding to the arrangement of the plurality of light emitting elements is read from the storage unit 32 to the illumination light distribution characteristic calculation unit 332. For example, in a case where the illumination unit 21 includes four light emitting elements and corresponding radiation angles $\theta_{E1}$, $\theta_{E2}$, $\theta_{E3}$, and $\theta_{E4}$ of the light emitting elements are calculated for a certain point of interest P, also values $\alpha(\theta_{E1}, \theta_{E2}, \theta_{E3}, \theta_{E4})$ in the radiation angle direction on the light distribution characteristics based on these radiation angles are calculated.

Referring back to FIG. 3, the depth gradient calculation unit 333 calculates a depth gradient on a point on the subject S on the basis of the depth image M (refer to FIG. 6) created by the depth image creation unit 331. The depth gradient is calculated by taking the derivative of the pixel value (namely, depth) of each of the pixels within the depth image. As illustrated in FIG. 4, the depth gradient gives a gradient (gradient angle $\theta$) of a contact surface on the point of interest P with respect to the surface orthogonal to the optical axis $Z_L$ of the collection optical system 22.

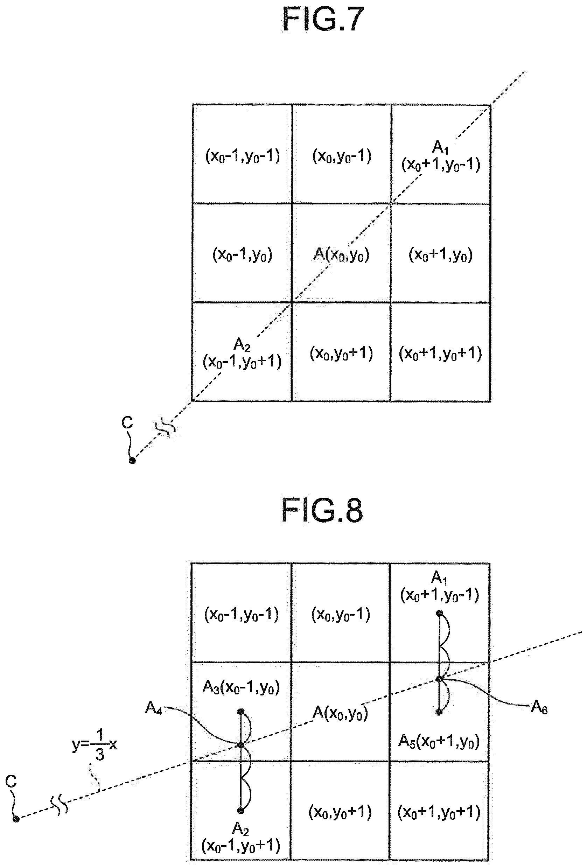

Now, a method for calculating the depth gradient by the depth gradient calculation unit 333 will be described in detail. FIGS. 7 and 8 are schematic diagrams for illustrating a method for calculating the gradient image. Rectangular regions illustrated in FIGS. 7 and 8 indicate the pixel of interest A $(x_0, y_0)$ and its peripheral pixels within the depth image M.

The depth gradient of the pixel of interest A is basically calculated using a pixel value (depth) of the pixel adjacent to the pixel of interest A on a line that connects a center C of the depth image M with the pixel of interest A. For example, as illustrated in FIG. 7, in a case where the centers of pixels $A_1$ and $A_2$ adjacent to the pixel of interest A are positioned on the line that connects the center C of the depth image M and the pixel of interest A, a depth gradient G on the pixel of interest A is given by the following formula (4), using vectors $CA_1$ and $CA_2$ directing from the center C to the pixels $A_1$ and $A_2$, respectively.

$$G = \tan^{-1}\left[\frac{Z(A_2) - Z(A_1)}{\sqrt{\left\{X\left(\overrightarrow{CA_2}\right) - X\left(\overrightarrow{CA_1}\right)\right\}^2 + \left\{Y\left(\overrightarrow{CA_2}\right) - Y\left(\overrightarrow{CA_1}\right)\right\}^2}}\right] \qquad \ldots (4)$$

In formula (4), a sign X( ) represents an x-component of a vector indicated in brackets, and a sign Y( ) represents a y-component of a vector indicated in brackets. Additionally, a sign Z( ) represents a pixel value namely, the depth, of the pixel indicated in brackets.

In contrast, as illustrated in FIG. 8, in a case where the center of pixel adjacent to the pixel of interest A is not positioned on the line that connects the center C of the depth image M and the pixel of interest A, the coordinate and the depth of the adjacent pixel are calculated by linear interpolation using peripheral pixels.

For example, suppose that the center C of the depth image M is the origin, and a line passing through the center C and the pixel of interest A is expressed as $y = (1/3)x$. In this case, vector $CA_4$ that gives coordinates of an intersection $A_4$ of a pixel in a column $(x_0 - 1)$ and a line $y = (1/3)x$ is calculated by formula (5-1) using vectors $CA_2$ and $CA_3$ respectively directed from the center C to pixels $A_2$ and $A_3$. Moreover, a depth $Z(A_4)$ at intersection $A_4$ is given by formula (5-2) using depths $Z(A_2)$, $Z(A_3)$ on the pixels $A_2$ and $A_3$.

$$\overrightarrow{CA_4} = \frac{2}{3}\overrightarrow{CA_3} + \frac{1}{3}\overrightarrow{CA_2} \qquad \qquad \ldots (5-1)$$

$$Z(A_4) = \frac{2}{3} Z(A_3) + \frac{1}{3}(A_2) \qquad \ldots (5\text{-}2)$$

Similarly, vector $CA_6$ that gives coordinates of an intersection $A_6$ of the pixel in the row $(x_0+1)$ and the line $y = (1/3)x$ is calculated by formula (6-1) using vectors $CA_1$ and $CA_5$, respectively directed from the center C to pixels $A_1$ and $A_5$. Moreover, a depth $Z(A_6)$ at the intersection $A_6$ is given by formula (6-2) using depths $Z(A_1)$, and $Z(A_6)$ on the pixels $A_1$, and $A_5$.

$$\overrightarrow{CA_6} = \frac{2}{3}\overrightarrow{CA_5} + \frac{1}{3}\overrightarrow{CA_1} \qquad \ldots (6\text{-}1)$$

$$Z(A_6) = \frac{2}{3} Z(A_5) + \frac{1}{3}(A_1) \qquad \ldots (6\text{-}2)$$

In this case, the depth gradient G of the pixel of interest A is calculated using the coordinates of the intersections $A_4$, and $A_6$ calculated by interpolation and the depth $Z(A_4)$ and $Z(A_6)$ at the intersections $A_4$ and $A_6$, similarly to formula (4).

In this manner, the depth gradient calculation unit 333 calculates the depth gradient on all the pixels within the depth image M.

Subsequently, on the basis of the depth gradient calculated by the depth gradient calculation unit 333, the reflected light distribution characteristic calculation unit 334 calculates a value in the reflection angle direction of the distribution characteristics of the illumination light reflected (that is, reflected light) from each point (for example, the point of interest P) on the subject S.

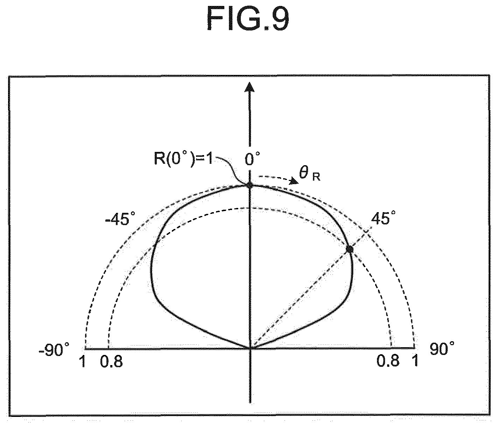

FIG. 9 is a schematic diagram illustrating exemplary light distribution characteristics of the reflected light. The light distribution characteristics of the reflected light refers to a reflectance in accordance with a reflection angle $\theta_R$ on the surface of the subject S. The light distribution characteristics illustrated in FIG. 9 has been normalized on the basis of reflectance $R(\theta_R=0)$ when the reflectance is maximum, that is, the reflection angle is $\theta_R = 0°$. The reflected light distribution characteristic calculation unit 334 reads a function or a table representing the light distribution characteristic illustrated in FIG. 9 from the storage unit 32, calculates the reflection angle $\theta_R$ from the relationship between the illumination light L1 incident from the illumination unit 21 to the point of interest P, and reflected light L2 emitted from the point of interest P to the collection optical system 22, and then, calculates a value $R(\theta_R)$ in the reflection angle direction on the light distribution characteristic by applying the function or the table representing the light distribution characteristic.

For example, when the reflection angle $\theta_R = 45°$, and when the value $R(45°)$ in the reflection angle direction on the light distribution characteristic is such that $R(45°) = 0.8$, the light intensity of the reflected light L2 radiated from the point of interest P in the direction of the image sensor 23 is 0.8 times the light intensity of the case where the reflection angle $\theta_R = 0°$.

Now, a method for calculating the reflection angle $\theta_R$ will be described. First, using a technique similar to the case of the illumination light distribution characteristic calculation unit 332, the reflected light distribution characteristic calculation unit 334 calculates a field angle $\phi$, viewed from the pixel A' on the light-receiving surface 23a, corresponding to the pixel of interest A (refer to FIG. 6) within the depth image M. Moreover, the depth gradient (gradient angle $\theta$) on the pixel of interest A is calculated from the depth gradient calculated by the depth gradient calculation unit 333. Then, the reflection angle $\theta_R$ is calculated from the field angle $\phi$ and a depth gradient (gradient angle $\theta$).

Referring back to FIG. 3, the luminance image creation unit 335 creates a luminance image having the luminance of the subject S image as a pixel value on the basis of the input image data. As illustrated in FIG. 2, since the ranging pixels 23c are sparsely arranged on the light-receiving surface 23a of the image sensor 23, image data has not been obtained at a pixel position in which one of the ranging pixels 23c is disposed. Accordingly, the luminance image creation unit 335 calculates, by interpolation, the luminance at the position of the ranging pixel 23c using image data based on the output value from the imaging pixel 23b located in the vicinity of the ranging pixel 23c.

Subsequently, the image plane illuminance calculation unit 336 calculates illuminance (image plane illuminance) on the image plane $E_f[lx]$ of the collection optical system 22 on the basis of the luminance image created by the luminance image creation unit 335. The image plane illuminance herein refers to the illuminance at the time when the reflected light L2 that has passed through the collection optical system 22 is incident into the image sensor 23 when the collection optical system 22 is considered to be an illumination system.

Image plane illuminance $E_f$ is given by the following formula (7) using an output value $V_{out}$ from the imaging pixel 23b (refer to FIG. 2) of the image sensor 23, a coefficient K, and exposure time t. The coefficient K is an ultimate coefficient that takes into account an absorption coefficient of the light on the imaging pixel 23b, a transform coefficient from electric charge to voltage, and the gain, loss, or the like, on a circuit including those for AD conversion and an amplifier. The coefficient K is predetermined by specifications of the image sensor 23. The image plane illuminance $E_f$ at a position of each of the ranging pixels 23c is calculated by interpolation using an output value $V_{out}$ from the imaging pixel 23b in the vicinity of the ranging pixel 23c.

$$E_f = V_{out} \times \frac{1}{K} \times \frac{1}{t} \qquad \ldots (7)$$

Subsequently, the object surface luminance calculation unit 337 calculates object surface luminance $L_S$ [cd/m$^2$] that is the luminance on the surface of the subject S on the basis of the image plane illuminance $E_f$. The object surface luminance $L_S$ is given by the following formula (8) using the image plane illuminance $E_f$, diameter D of the collection optical system 22, focal length b, and intensity transmittance T(h).

$$L_S = E_f \times \frac{4}{\pi} \times \frac{b^2}{D^2} \times \frac{1}{T(h)} \qquad \ldots (8)$$

Subsequently, the irradiation illuminance calculation unit 338 calculates irradiation illuminance $E_0$[lx] of the illumination light L1 emitted on the subject S, on the basis of the object surface luminance $L_S$. By being reflected from the point of interest P of the subject S, the illumination light L1 is attenuated by the reflectance $R_0$ on the surface of the subject S, while being attenuated by the light distribution characteristic in accordance with the reflection angle $\theta_R$. Accordingly, the irradiation illuminance $E_0$ can be obtained by backward calculation by the following formula (9) using the object surface luminance $L_S$, the reflectance $R_0$ of the subject S, and the value $R(\theta_R)$ in the reflection angle direction of the distribution characteristics of the reflected light L2 calculated by the reflected light distribution characteristic calculation unit 334.

$$E_0 = L_S \times \frac{\pi}{R_0 \cdot R(\theta_R)} \qquad \ldots (9)$$

The reflectance $R_0$ is a value determined in accordance with the surface property of the subject S and stored in the storage unit 32 beforehand. The storage unit 32 may store a plurality of values of the reflectance $R_0$ corresponding to the types of subject to be observed such as gastric and colonic mucosa. In this case, the irradiation illuminance calculation unit 338 uses the reflectance $R_0$ that is selected in accordance with the signal input from the operation input unit 35 (refer to FIG. 1) .

The irradiation illuminance $E_0$ calculated in this manner represents the illuminance level obtained by the process in which the illumination light L1 emitted from the illumination unit 21 reached the point of interest P of the subject S. During this process, the illumination light L1 emitted from the illumination unit 21 is attenuated by the value $\alpha(\theta_E)$ in the radiation angle direction on the light distribution characteristic in accordance with an irradiation distance $d_L$ to the point of interest P, and the radiation angle $\theta_E$. Accordingly, the relationship of the following formula (10) is established between luminance $L_{LED}$ of the illumination unit 21 and the irradiation illuminance $E_0$ on the point of interest P.

$$E_0 = \frac{4 \cdot \alpha(\theta_E) \cdot L_{LED} \cdot S_{LED} \cdot Em_{SPE}}{d_L^2} \qquad \ldots (10)$$

In formula (10), a sign $S_{LED}$ represents a surface area of a region onto which the illumination light L1 is emitted from the illumination unit 21. Moreover, a sign $Em_{SPE}$ represents a spectral characteristic coefficient of the illumination light L1.

Then, the irradiation distance calculation unit 339 obtains, from the illumination light distribution characteristic calculation unit 332, the value $\phi(\theta_E)$ in the radiation angle direction of the distribution characteristics of the illumination light, and calculates the irradiation distance $d_L$[m] given by the following formula (11) using the value $\alpha(\theta_E)$ in the radiation angle direction on the light distribution characteristic, and the irradiation illuminance $E_0$.

$$d_L = \sqrt{\frac{4 \cdot \alpha(\theta_E) \cdot L_{LED} \cdot S_{LED} \cdot Em_{SPE}}{E_0}} \qquad \ldots (11)$$

Subsequently, the subject distance calculation unit 340 calculates a subject distance $d_S$[m] obtained by projecting the irradiation distance $d_L$ onto the optical axis $Z_L$ by the following formula (12) using the radiation angle $\theta_E$.

$$d_S = d_L \cdot \cos\theta_E \qquad \ldots (12)$$

The depth calculation unit 33b executes the above-described sequential processing on each of the pixels within the depth image M, creates a distance map that associates the calculated subject distance $d_S$ to each of the pixels within an image for display, created by the image processing unit 33a, and then, stores the distance map in the storage unit 32. These processes complete processing onto the image data and ranging data obtained from the imaging unit 2.

As described above, according to the first embodiment, a depth image is created on the basis of the ranging data measured by the ranging pixel 23c while the depth gradient is calculated, a value in the radiation angle direction of the distribution characteristics of the illumination light and a value in the reflection angle direction of the distribution characteristics of the reflected light are individually calculated on the basis of the calculated depth image and the depth gradient, and a subject distance is calculated from the luminance of the image using these light distribution characteristic values. Accordingly, it is possible to drastically enhance the accuracy of the subject distance compared with the case where the light distribution characteristic value is not used.

Moreover, according to the first embodiment, the ranging data are obtained from the ranging pixels 23c sparsely arranged on the light-receiving surface 23a of the image sensor 23. Accordingly, it is possible to drastically reduce the data processing amount on the image sensor 23 and data communication amount from the imaging unit 2 to the image processing apparatus 3. Accordingly, this makes it possible to suppress reduction of the imaging frame rate on the image sensor 23.

(Modification)

In the above-described first embodiment, the image plane phase difference detection AF sensor in which the plurality of imaging pixels 23b and the plurality of ranging pixels 23c are arranged on the same light-receiving surface 23a, is employed as the image sensor 23. However, the configuration of the image sensor 23 is not limited to this. For example, a typical imaging element, such as a CMOS and CCD, may be used in combination with a TOF-system ranging sensor.

(Second Embodiment)

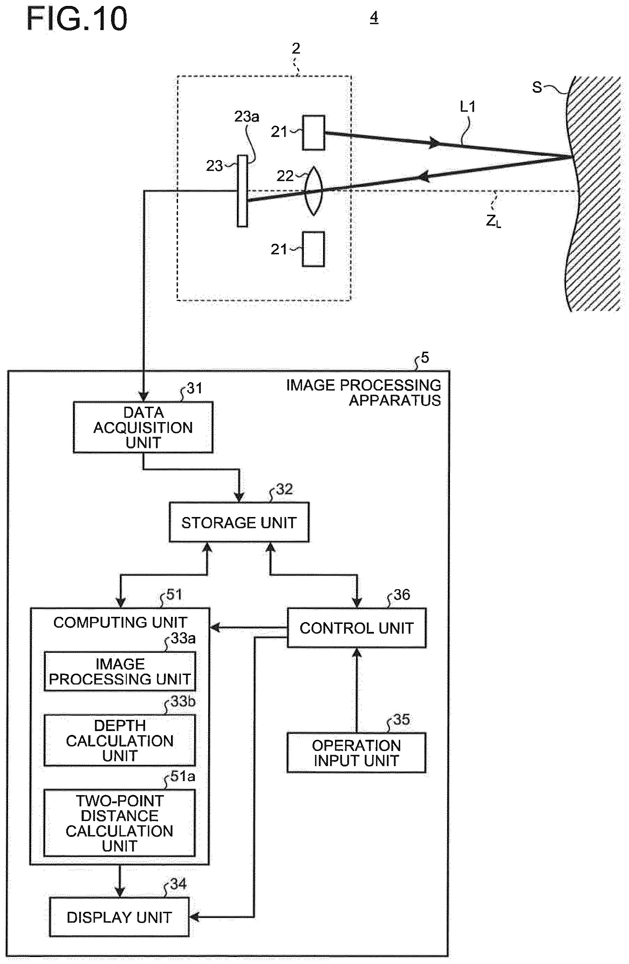

Next, a second embodiment of the present invention will be described. FIG. 10 is a block diagram illustrating a configuration of a ranging system according to a second embodiment of the present invention. As illustrated in FIG. 10, a ranging system 4 according to the second embodiment includes an image processing apparatus 5 instead of the image processing apparatus 3 illustrated in FIG. 1. The configuration and operation of the imaging unit 2 is similar to the case of the first embodiment.

The image processing apparatus 5 includes, instead of the computing unit 33 illustrated in FIG. 1, a computing unit 51 further including a two-point distance calculation unit 51a. The configuration and operation of each element of the image processing apparatus 5 other than the computing unit 51, and operation of the image processing unit 33a and the depth calculation unit 33b included in the computing unit 51, are similar to those of the first embodiment.

On an image for display of the subject S created by the image processing unit 33a, the two-point distance calculation unit 51a calculates a distance between two points designated by a signal input from the operation input unit 35.

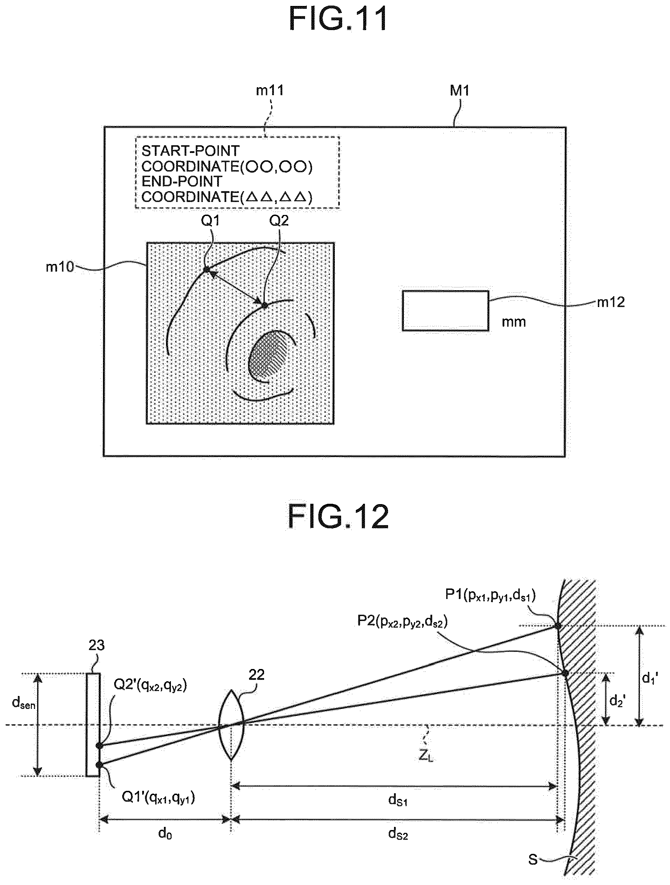

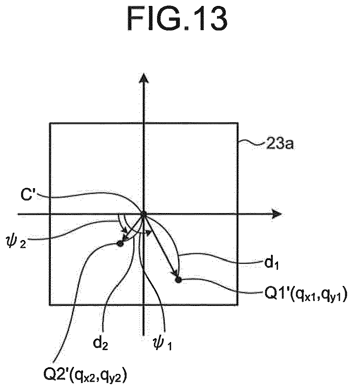

Next, a method for measuring the distance on the subject S, corresponding to a distance between the two points within the image will be described with reference to FIGS. 11 to 13. FIG. 11 is a schematic diagram illustrating an exemplary screen displayed on the display unit 34. FIGS. 12 and 13 are schematic diagrams for illustrating a principle of measuring the distance between two points. Hereinafter, a distance map related to the subject S (refer to the first embodiment) has been created and stored in the storage unit 32.

First, as illustrated in FIG. 11, the control unit 36 displays, onto the display unit 34, a screen M1 including an image m10 for display of the subject S created by the image processing unit 33a. The screen M1 includes, in addition to the image m10, a coordinate display field m11 that displays coordinates of any two points (start point and end point) selected on the image m10 by a user, and includes a distance display field m12 that displays a distance between the two points on the subject S corresponding to any two points selected on the image m10 by the user.

When any two points Q1 and Q2 on the image m10 are designated by predetermined pointer operation (e.g. click operation) onto the screen M1 using the operation input unit 35, the operation input unit 35 inputs coordinate values of the two designated points Q1 and Q2 on the image m10 into the control unit 36.

As described above, the distance map related to the subject S has already been obtained, and therefore, the distance from a point on the subject S corresponding to each of the pixel positions on the image m10, to the imaging unit 2, is known. Moreover, as illustrated in FIG. 12, a sensor size $d_{sen}$ and a distance $d_0$ from the collection optical system 22 to the light-receiving surface 23a are given as design values.

Accordingly, the two-point distance calculation unit 51a obtains coordinate values of the two points Q1 and Q2 on the image m10 from the control unit 36, reads the distance map from the storage unit 32, and obtains distances $d_{s1}$ and $d_{s2}$ from two points P1 and P2 on the subject S, corresponding to these two points Q1 and Q2 to the imaging unit 2 (collection optical system 22).

Moreover, as illustrated in FIG. 13, the two-point distance calculation unit 51a obtains coordinate values ($q_{x1}$, $q_{y1}$) and ($q_{x2}$, $q_{y2}$) of two points Q1' and Q2' on the light-receiving surface 23a of the image sensor 23, corresponding to two points Q1 and Q2 on the image m10, and then, calculates image heights $d_1$ and $d_2$ (distance from the optical axis $Z_L$) using the obtained coordinate values, the sensor size $d_{sen}$, and the distance $d_0$. The coordinate values ($q_{x1}$, $q_{y1}$) and ($q_{x2}$, $q_{y2}$) are coordinates when a point C' on the light-receiving surface 23a, on which the optical axis $Z_L$ passes through, is defined as the origin.

Furthermore, the two-point distance calculation unit 51a obtains rotation angles $\psi_1$ and $\psi_2$ from predetermined axes for the vectors respectively directing from the point C' to the points Q1' and Q2'.

Subsequently, the two-point distance calculation unit 51a calculates heights $d_1'$ and $d_2'$ of the subject (distance from the optical axis $Z_L$) at the points P1 and P2, respectively, on the basis of the image heights $d_1$ and $d_2$, the distance $d_1$ from the collection optical system 22 to the light-receiving surface 23a, and the distances $d_{s1}$ and $d_{s2}$ from the points P1 and P2 on the subject S to the collection optical system 22.

When the rotation angles $\psi_1$ and $\psi_2$ and the heights of the subject $d_1'$ and $d_2'$ illustrated in FIG. 13 are used, the coordinates ($p_{x1}$, $p_{y1}$, $d_{S1}$) and ($p_{x2}$, $p_{y2}$, $d_{S2}$) of the points P1 and P2 on the subject S are respectively given by the following formulas (13) and (14).

$$(p_{x1}, p_{y1}, d_{S1}) = (d_1' \cos\psi_1, d_1' \sin\psi_1, d_{S1}) \qquad \ldots (13)$$

$$(p_{x2}, p_{y2}, d_{S2}) = (d_2' \cos\psi_2, d_2' \sin\psi_2, d_{S2}) \qquad \ldots (14)$$

The two-point distance calculation unit 51a calculates a distance d between these coordinates ($p_{x1}$, $p_{y1}$, $d_{S1}$) and ($p_{x2}$, $p_{y2}$, $d_{S2}$), outputs the result to the display unit 34 to be displayed, for example, in a distance display field m12 of the screen M1. The distance d may be a distance on a surface orthogonal to the optical axis $Z_L$ calculated from two-dimensional coordinates ($p_{x1}$, $p_{y1}$) and ($p_{x2}$, $p_{y2}$), or may be a distance in a three-dimensional space, calculated from three-dimensional coordinates ($p_{x1}$, $p_{y1}$, $d_{S1}$) and ($p_{x2}$, $p_{y2}$, $d_{S2}$).

As described above, according to the second embodiment of the present invention, it is possible to accurately calculate the distance between the two points on the subject S, corresponding to any two points designated on the image m10, by using the distance map associated with each of the pixels within the image m10.

(Third Embodiment)

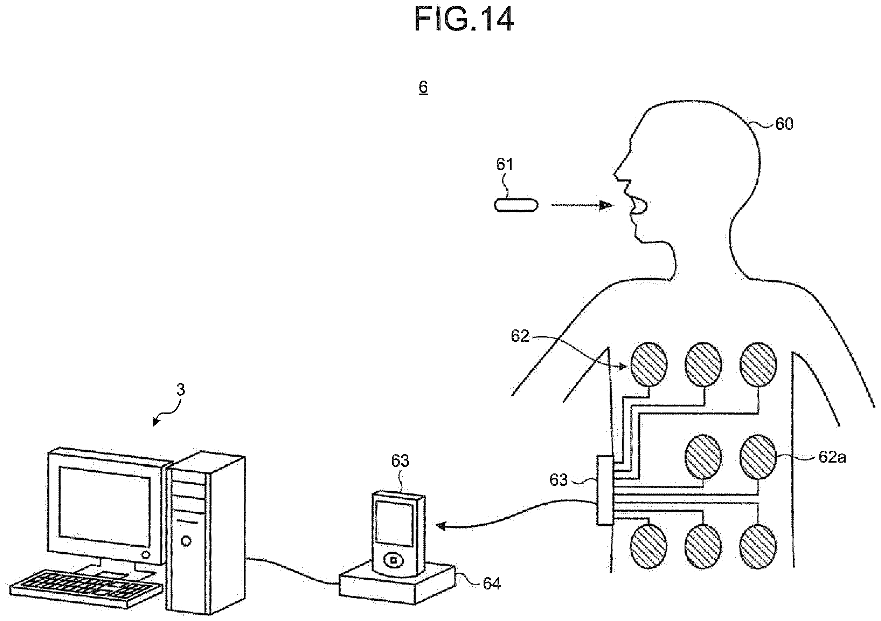

Next, a third embodiment of the present invention will be described. FIG. 14 is a schematic diagram illustrating a configuration of an endoscope system according to the third embodiment of the present invention. As illustrated in FIG. 14, an endoscope system 6 according to the third embodiment includes: a capsule endoscope 61 that is introduced into a subject 60, performs imaging, generates an image signal, and wireless transmits the image signal; a receiving device 63 that receives the image signal wirelessly transmitted from the capsule endoscope 61 via a receiving antenna unit 62 attached on the subject 60; and the image processing apparatus 3. The configuration and operation of the image processing apparatus 3 are similar to the case of the first embodiment (refer to FIG. 1). The image processing apparatus 3 obtains image data from the receiving device 63, performs predetermined image processing on the data, and displays an image within the subject 60. Alternatively, the image processing apparatus 5 according to the second embodiment may be employed instead of the image processing apparatus 3.

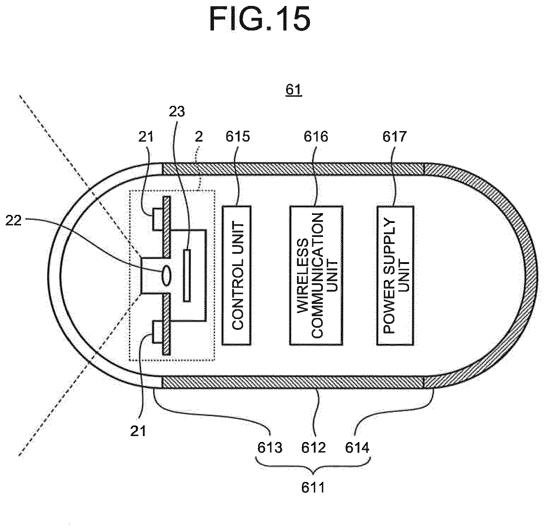

FIG. 15 is a schematic diagram illustrating an exemplary configuration of the capsule endoscope 61. The capsule endoscope 61 is introduced into the subject 60 by oral ingestion, or the like, moves along the gastrointestinal tract, and is finally discharged to the outside from the subject 60. During that period, while moving inside the organ (gastrointestinal tract) by peristaltic motion, the capsule endoscope 61 sequentially generates image signals by imaging inside the subject 60, and wirelessly transmits the image signals.

As illustrated in FIG. 15, the capsule endoscope 61 includes a capsule-shaped casing 611 configured to contain the imaging unit 2 including the illumination unit 21, the collection optical system 22, and the image sensor 23. The capsule-shaped casing 611 is an outer casing formed into a size that can be introduced into the inside of the organ of the subject 60. Moreover, the capsule-shaped casing 611 includes a control unit 615, a wireless communication unit 616, and a power supply unit 617. The control unit 615 controls each element of the capsule endoscope 61. The wireless communication unit 616 wirelessly transmits the signal processed by the control unit 615 to the outside of the capsule endoscope 61. The power supply unit 617 supplies power to each element of the capsule endoscope 61.

The capsule-shaped casing 611 includes a cylindrical casing 612 and dome-shaped casings 613 and 614, being implemented by closing both ends of the openings of the cylindrical casing 612 by the dome-shaped casings 613 and 614. The cylindrical casing 612 and the dome-shaped casing 614 is a casing substantially opaque for the visible light. In contrast, the dome-shaped casing 613 is an optical member having a dome-like shape, transparent to the light having a predetermined wavelength band, such as visible light. The capsule-shaped casing 611 configured in this manner contains, using fluid-tight sealing, the imaging unit 2, the control unit 615, the wireless communication unit 616, and the power supply unit 617.

The control unit 615 controls operation of each element of the capsule endoscope 61 and controls input and output of signals between these elements. Specifically, the control unit 615 controls imaging frame rate of the image sensor 23 of the imaging unit 2, and causes the illumination unit 21 to emit light in synchronization with the imaging frame rate. Moreover, the control unit 615 performs predetermined signal processing on an image signal output from the image sensor 23 and wirelessly transmits the image signal from the wireless communication unit 616.

The wireless communication unit 616 obtains an image signal from the control unit 615, generates a wireless signal by performing modulating processing, or the like, on the image signal, and transmits the processed signal to the receiving device 63.

The power supply unit 617 is a power storage unit such as a button cell battery and a capacitor, and supplies power to each element of the capsule endoscope 61 (imaging unit 2, control unit 615, and wireless communication unit 616).

Referring back to FIG. 14, the receiving antenna unit 62 includes a plurality of (eight in FIG. 14) receiving antennas 62a. Each of the receiving antennas 62a is implemented by a loop antenna, for example, and disposed at a predetermined position on an external surface of the subject 60 (for example, position corresponding to individual organs inside the subject 60, that is, passage region of the capsule endoscope 61).

The receiving device 63 receives an image signal wirelessly transmitted from the capsule endoscope 61 via these receiving antennas 62a, performs predetermined processing on the receiving image signal, and stores the image signal and its related information on an internal memory. The receiving device 63 may include a display unit that displays receiving states of the image signal wirelessly transmitted from the capsule endoscope 61, and an input unit including an operation button to operate the receiving device 63. The image signal stored in the receiving device 63 is transmitted to the image processing apparatus 3 by setting the receiving device 63 on a cradle 64 connected to the image processing apparatus 3.

(Fourth Embodiment)

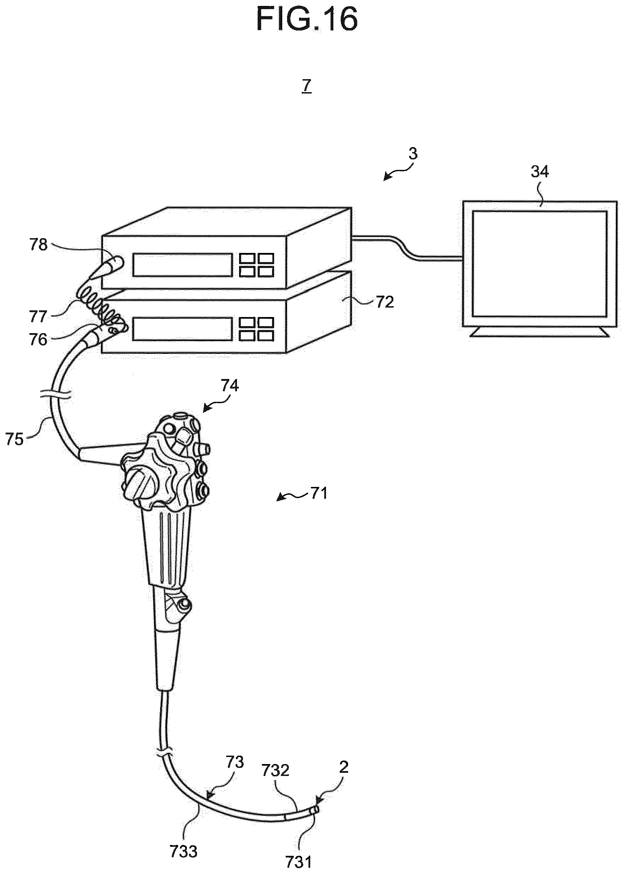

Next, a fourth embodiment of the present invention will be described. FIG. 16 is a schematic diagram illustrating a configuration of an endoscope system according to the fourth embodiment of the present invention. As illustrated in FIG. 16, an endoscope system 7 according to the fourth embodiment includes: an endoscope 71 that is introduced into the body of the subject, performs imaging, creates and outputs an image; a light source apparatus 72 that generates illumination light to be emitted from the distal end of the endoscope 71; and an image processing apparatus 3. The configuration and operation of the image processing apparatus 3 are similar to the case of the first embodiment (refer to FIG. 1). The image processing apparatus 3 obtains image data generated by the endoscope 71, performs predetermined image processing on the data, and displays an image within the subject on the display unit 34. Alternatively, the image processing apparatus 5 according to the second embodiment may be employed instead of the image processing apparatus 3.

The endoscope 71 includes an insertion unit 73 that is a flexible and elongated portion, an operating unit 74 that is connected on a proximal end of the insertion unit 73 and receives input of various operation signals, and a universal cord 75 that extends from the operating unit 74 in a direction opposite to the extending direction of the insertion unit 73, and incorporates various cables for connecting with the image processing apparatus 3 and the light source apparatus 72.

The insertion unit 73 includes a distal end portion 731, a bending portion 732 that is a bendable portion formed with a plurality of bending pieces, and a flexible needle tube 733 that is a long and flexible portion connected with a proximal end of the bending portion 45. At the distal end portion 731 of the insertion unit 73, the imaging unit 2 (refer to FIG. 1) is provided. The imaging unit 2 includes the illumination unit 21 that illuminates the inner portion of the subject by illumination light generated by the light source apparatus 72, the collection optical system 22 that collects illumination light reflected within the subject, and the image sensor 23.

Between the operating unit 74 and the distal end portion 731, a cable assembly and a light guide for transmitting light are connected. The cable assembly includes a plurality of signal lines arranged in a bundle, to be used for transmission and reception of electrical signals with the image processing apparatus 3. The plurality of signal lines includes a signal line for transmitting an image signal output from the image element to the image processing apparatus 3, and a signal line for transmitting a control signal output from the image processing apparatus 3 to the imaging element.

The operating unit 74 includes a bending knob, a treatment tool insertion section, and a plurality of switches. The bending knob is provided for bending the bending portion 732 in up-down directions, and in right- and-left directions. The treatment tool insertion section is provided for inserting treatment tools such as a biological needle, biopsy forceps, a laser knife, and an examination probe. The plurality of switches is used for inputting operating instruction signals into peripheral devices such as the image processing apparatus 3 and the light source apparatus 72.

The universal cord 75 incorporates at least a light guide and a cable assembly. Moreover, the end portion of the side differing from the side linked to the operating unit 74 of the universal cord 75 includes a connector unit 76 that is removably connected with the light source apparatus 72, and includes an electrical connector unit 78 that is electrically connected with the connector unit 76 via a coil cable 77 having a coil shape, and is removably connected with the image processing apparatus 3. The image signal output from the imaging element is input into the image processing apparatus 3 via the coil cable 77 and the electrical connector unit 78.

The first to fourth embodiments of the present invention have been described hereinabove merely as examples for implementation of the present invention, and thus, the present invention is not intended to be limited to these embodiments. Furthermore, in the present invention, a plurality of elements disclosed in the above-described first to fourth embodiments may be appropriately combined to form various inventions. The present invention can be modified in various manners in accordance with the specification, or the like, and it is apparent from the description given above that various other embodiments can be implemented within the scope of the present invention. Reference Signs List

1, 4 ranging system
2 imaging unit
3, 5 image processing apparatus
7 endoscope system
21 illumination unit
22 collection optical system
23 image sensor
23a light-receiving surface
23b imaging pixel
23c ranging pixel
23d signal processing circuit
31 data acquisition unit
32 storage unit
33, 51 computing unit
33a image processing unit
33b depth calculation unit
34 display unit
35 operation input unit
36 control unit
51a two-point distance calculation unit
60 subject
61 capsule endoscope
62 receiving antenna unit
62a receiving antenna
63 receiving device
64 cradle
71 endoscope
72 light source apparatus
73 insertion unit

74 operating unit
75 universal cord
76 connector unit
77 coil cable
78 electrical connector unit
331 depth image creation unit
332 illumination light distribution characteristic calculation unit
333 depth gradient calculation unit
334 reflected light distribution characteristic calculation unit
335 luminance image creation unit
336 image plane illuminance calculation unit
337 object surface luminance calculation unit
338 irradiation illuminance calculation unit
339 irradiation distance calculation unit
340 subject distance calculation unit
611 capsule-shaped casing
612 cylindrical casing
613, 614 dome-shaped casing
615 control unit
616 wireless communication unit
617 power supply unit
731 distal end portion
732 bending portion
733 flexible needle tube

**Claims**

1. An image processing apparatus for performing image processing based on image data and ranging data output from an imaging unit, the imaging unit comprising: an illumination unit configured to generate illumination light to be emitted onto a subject; a collection optical system configured to collect the illumination light reflected from the subject; and an image sensor configured to receive the illumination light collected by the collection optical system, and output the image data representing an image of the subject and output the ranging data representing a distance to the subject, based on the illumination light, the image processing apparatus comprising:

   an illumination light distribution characteristic calculation unit configured to calculate a parameter of the illumination light emitted onto a point on the subject, based on the ranging data;
   a reflected light distribution characteristic calculation unit configured to calculate a parameter of reflected light of the illumination light reflected from the subject, based on a gradient of a depth on the point on the subject calculated from the ranging data; and
   a subject distance calculation unit configured to calculate the distance between the imaging unit and the subject in an optical axis direction of the collection optical system, based on the image data, the parameter of the illumination light, and the parameter of the reflected light.

2. The image processing apparatus according to claim 1, further comprising
   a depth image creation unit configured to create, based on the ranging data, a depth image in which the depth to the point on the subject corresponding to each of pixel positions of an image of the subject created based on the image data is defined as a pixel value of each pixel,
   wherein the illumination light distribution characteristic calculation unit is configured to calculate a value of distribution characteristics of the illumination light in a radiation angle direction, based on the depth image.

3. The image processing apparatus according to claim 2, wherein the depth image creation unit is configured to perform interpolation on the depth at a pixel position where the ranging data has not been obtained, among the pixel positions of the image of the subject, using the ranging data at a pixel position where the ranging data has been obtained.

4. The image processing apparatus according to claim 2, further comprising a depth gradient calculation unit configured to calculate the gradient of the depth for each of the pixel positions of the image of the subject, based on the depth calculated by the depth image creation unit,

wherein the reflected light distribution characteristic calculation unit is configured to calculate a value of distribution characteristics of the reflected light in a reflection angle direction, based on the gradient of the depth calculated by the depth gradient calculation unit.

5. The image processing apparatus according to claim 1, further comprising:

an image processing unit configured to create an image for display based on the image data;
a display unit configured to display the image for display;
an operation input unit configured to input a signal for designating any two points on the image for display; and
a two-point distance calculation unit configured to calculate a distance between two points on the subject corresponding to any two points on the image for display.

6. A ranging system comprising:

the image processing apparatus according to claim 1; and
the imaging unit.

7. An endoscope system comprising:

the image processing apparatus according to claim 1; and
a capsule endoscope comprising the imaging unit and a capsule-shaped casing that accommodates the imaging unit.

8. An endoscope system comprising:

the image processing apparatus according to claim 1; and
a video scope comprising: an insertion unit configured to be inserted into the subject; and the imaging unit at a distal end portion of the insertion unit.

# FIG.1

<u>1</u>

2

23a

21

23

22

23

S

L1

$Z_L$

21

3

IMAGE PROCESSING
APPARATUS

31

DATA
ACQUISITION
UNIT

32

STORAGE UNIT

33

COMPUTING UNIT

36

CONTROL UNIT

33a

IMAGE
PROCESSING UNIT

33b

DEPTH
CALCULATION
UNIT

35

OPERATION
INPUT UNIT

34

DISPLAY UNIT

# FIG.2

SIGNAL PROCESSING
CIRCUIT 23d

TO IMAGE PROCESSING
APPARATUS

# FIG.3

IMAGE DATA                                    RANGING DATA

33b

DEPTH CALCULATION
UNIT

335                                           331

LUMINANCE IMAGE                               DEPTH IMAGE
CREATION UNIT                                 CREATION UNIT

336

IMAGE PLANE
ILLUMINANCE
CALCULATION UNIT

337                                           333                    332

OBJECT SURFACE          DEPTH GRADIENT        ILLUMINATION LIGHT
LUMINANCE               CALCULATION UNIT      DISTRIBUTION
CALCULATION UNIT                              CHARACTERISTIC
                                              CALCULATION UNIT

338                     334

IRRADIATION             REFLECTED LIGHT
ILLUMINANCE             DISTRIBUTION
CALCULATION UNIT        CHARACTERISTIC
                        CALCULATION UNIT

339

IRRADIATION
DISTANCE
CALCULATION UNIT

340

SUBJECT DISTANCE
CALCULATION UNIT

# FIG.4

# FIG.5

# FIG.6

# FIG.7

| | | |
|---|---|---|
| $(x_0-1,y_0-1)$ | $(x_0,y_0-1)$ | $A_1$ $(x_0+1,y_0-1)$ |
| $(x_0-1,y_0)$ | $A(x_0,y_0)$ | $(x_0+1,y_0)$ |
| $A_2$ $(x_0-1,y_0+1)$ | $(x_0,y_0+1)$ | $(x_0+1,y_0+1)$ |

C

# FIG.8

| | | |
|---|---|---|
| $(x_0-1,y_0-1)$ | $(x_0,y_0-1)$ | $A_1$ $(x_0+1,y_0-1)$ |
| $A_3(x_0-1,y_0)$ | $A(x_0,y_0)$ | $A_5(x_0+1,y_0)$ |
| $A_2$ $(x_0-1,y_0+1)$ | $(x_0,y_0+1)$ | $(x_0+1,y_0+1)$ |

$A_4$

$A_6$

$y=\dfrac{1}{3}x$

C

# FIG.9

# FIG.10

4

2

23a

21

L1

S

23

22

23

21

Z_L

5

IMAGE PROCESSING
APPARATUS

31

DATA
ACQUISITION
UNIT

32

STORAGE UNIT

51

COMPUTING UNIT

33a

IMAGE
PROCESSING UNIT

33b

DEPTH
CALCULATION
UNIT

51a

TWO-POINT
DISTANCE
CALCULATION
UNIT

36

CONTROL UNIT

35

OPERATION
INPUT UNIT

34

DISPLAY UNIT

# FIG.11

# FIG.12

# FIG.13

FIG.14

EP 3 318 173 A1

# FIG.15

# FIG.16

## INTERNATIONAL SEARCH REPORT

| International application No. |
| --- |
| PCT/JP2016/054621 |

A. CLASSIFICATION OF SUBJECT MATTER
*A61B1/00*(2006.01)i, *A61B1/04*(2006.01)i, *G01S17/89*(2006.01)i, *G02B23/24* (2006.01)i

According to International Patent Classification (IPC) or to both national classification and IPC

B. FIELDS SEARCHED

Minimum documentation searched (classification system followed by classification symbols)
A61B1/00-1/32, G01S17/89, G02B23/24-23/26, G03B35/00-37/06, H04N5/222-5/257, A61B5/103-5/113

Documentation searched other than minimum documentation to the extent that such documents are included in the fields searched
Jitsuyo Shinan Koho          1922-1996   Jitsuyo Shinan Toroku Koho   1996-2016
Kokai Jitsuyo Shinan Koho    1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2015-119277 A  (Olympus Imaging Corp.), 25 June 2015 (25.06.2015), paragraphs [0062] to [0063], [0073] to [0075]; fig. 9 (Family: none) | 1-8 |
| A | JP 2002-65585 A  (Fuji Photo Film Co., Ltd.), 05 March 2002 (05.03.2002), paragraphs [0010] to [0025]; fig. 2 (Family: none) | 1-8 |
| A | JP 2009-204991 A  (Funai Electric Co., Ltd.), 10 September 2009 (10.09.2009), paragraph [0027]; fig. 3 (Family: none) | 1-8 |

☐ Further documents are listed in the continuation of Box C.      ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    09 May 2016 (09.05.16) | Date of mailing of the international search report<br>    17 May 2016 (17.05.16) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br><br>Telephone No. |

Form PCT/ISA/210 (second sheet) (January 2015)

**EP 3 318 173 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013232751 A
- JP 2009267436 A
- JP 2009041929 A